# EUROPÄISCHE PATENTSCHRIFT

(11) **EP 1 015 421 B1**
(45) Veröffentlichungstag und Bekanntmachung des Hinweises auf die Patenterteilung: **31.03.2004**
(21) Anmeldenummer: 96943916.5
(22) Anmeldetag: 11.12.1996
(51) Int. Cl.: C07C 257/18, C07D 211/06, A61K 31/155, A61K 31/445

(54) **NEUE PHENYLAMIDINDERIVATE, VERFAHREN ZU IHRER HERSTELLUNG UND IHRE VERWENDUNG ALS ARZNEIMITTEL**
NEW PHENYLAMIDINE DERIVATIVES, A PROCESS FOR PREPARING THE SAME AND THEIR USE AS MEDICAMENTS
NOUVEAUX DERIVES DE PHENYLAMIDINE, LEUR PROCEDE DE PREPARATION ET LEUR UTILISATION COMME MEDICAMENTS

(30) Priorität: 13.12.1995 DE 19546452
(43) Veröffentlichungstag der Anmeldung: 05.07.2000
(73) Patentinhaber: Boehringer Ingelheim Pharma GmbH & Co.KG, 55218 Ingelheim am Rhein (DE)
(72) Erfinder: ANDERSKEWITZ, Ralf, D-88471 Laupheim (DE); SCHROMM, Kurt, D-55218 Ingelheim (DE); RENTH, Ernst-Otto, D-24105 Kiel (DE); BIRKE, Franz, D-55218 Ingelheim (DE); JENNEWEIN, Hans, D-65193 Wiesbaden (DE); MEADE, Christopher, D-55411 Bingen (DE); DING, Andreas, D-88400 Biberach (DE)
(74) Vertreter: Laudien, Dieter, Dr.
(86) Internationale Anmeldenummer: PCT/EP1996/005529
(87) Internationale Veröffentlichungsnummer: WO 1997/021670

(56) Entgegenhaltungen:
- WO-A-93/16036
- DE-A- 4 309 285
- US-A- 5 246 965

## Beschreibung

Die Erfindung betrifft neue Phenylamidinderivate, Verfahren zu ihrer Herstellung sowie ihre Verwendung als Arzneimittel.

Aus dem US Patent US 5,246,965, der internationalen Patentanmeldung WO 93/16036 und der deutschen Patentanmeldung DE 43 09 285 sind ähnliche Phenylamidinderivate mit LTB₄ antagonistischer Wirkung bekannt.

Der Erfindung lag im Hinblick auf diese Dokumente die Aufgabe zu Grunde neue LTB₄ Antagonisten mit verbesserten Eigenschaften, die insbesondere oral zu verabreichen sind, zur Verfügung zu stellen.

Die erfindungsgemäßen Phenylamidine entsprechen der allgemeinen Formel I 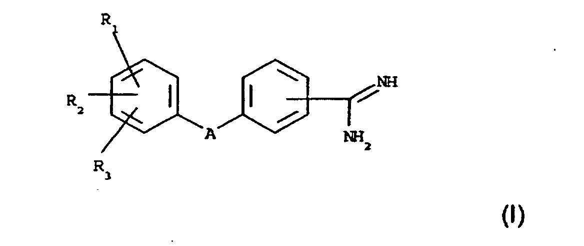 worin
- A: X₁-CₘH₂ₘ-X₂-, m eine ganze Zahl 2 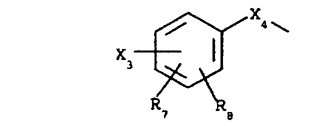 und
- X₁: O;
- X₂: 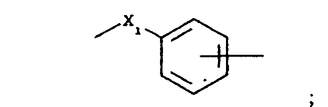
- X₃: -X₁-CₙH₂ₙ-, worin n eine ganze Zahl 1 oder 2;
- X₄: -CₙH₂ₙ-X₁-, worin n eine ganze Zahl 1 oder 2;
- R₁: CR₄R₅Ar₃, C(CH₃)₂R₆;
- R₂: H, C₁-C₆-Alkyl, OH, Cl, O-(C₁-C₆)-Alkyl;
- R₃: H, C₁-C₆-Alkyl;
- R₄: C₁-C₄-Alkyl, CF₃, CH₂OH;
- R₅: H, C₁-C₄-Alkyl, CF₃, CH₂OH; oder
- R₄ und R₅: auch gemeinsam eine C₄-C₆-Alkylengruppe bilden können;
- R₆: CH₂OH, COOH, COO(C₁-C₄)-Alkyl, CONR₉R₁₀, CH₂NR₉R₁₀;
- R₇: H,F, Cl, Br, OH, C₁-C₆-Alkyl oder C₁-C₆-Alkoxy;
- R₈: H, F,Cl, Br, OH, C₁-C₆-Alkyl oder C₁-C₆-Alkoxy;
- R₉: H, C₁-C₆-Alkyl;
- R₁₀: H oder C₁-C₆-Alkyl, oder
- R₉ und R₁₀: gemeinsam auch eine C₄-C₆-Alkylengruppe darstellen können;

Ar₃ einen gegebenenfalls durch ein oder mehrere Niederalkylgruppen mit 1 bis 4 Kohlenstoffatomen, Trifluormethylgruppen, Cyanogruppen, Alkoxygruppen mit 1 bis 8 Kohlenstoffatomen, Nitrogruppen, Hydroxygruppen, Aminogruppen und/oder einem oder mehreren Halogenatomen substituierten Arylrest mit 6 Kohlenstoffatomen
bedeuten können - mit der Maßgabe, daß
- R₁: nicht die Bedeutung eines über eine C₁-C₄-Alkyleneinheit gebundenen unsubstituierten Phenylrestes haben darf;
gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren.

Bevorzugt sind Verbindungen der allgemeinen Formel I, worin
- A: 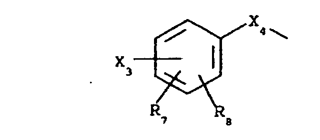 und
- X₁: O;
- X₃: -X₁-CH₂-;
- X₄: -CH₂-X₁-;
- R₁: CR₄R₅Ar₃, C(CH₃)₂R₆;
- R₂: H, OH, O-(C₁-C₆)-Alkyl;
- R₃: H;
- R₄: CH₃, CH₂OH;
- R₅: H, CH₃, CH₂OH; oder
- R₄ und R₅: auch gemeinsam eine C₄-C₆-Alkylengruppe bilden können;
- R₆: CH₂OH, COOH, COO(C₁-C₄)-Alkyl, CONR₉R₁₀, CH₂NR₉R₁₀;
- R₇: H;
- R₈: H;
- R₉: H, C₁-C₆-Alkyl;
- R₁₀: H oder C₁-C₆-Alkyl; oder
- R₉ und R₁₀: gemeinsam auch eine C₄-C₆-Alkylengruppe darstellen können;
- Ar₃: einen gegebenenfalls ein- oder mehrfach mit Hydroxy oder mit Hydroxy und C₁-C₄-Alkyl substituierten Arylrest
bedeuten können - gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren.

Soweit nicht im einzelnen abweichende Angaben gemacht werden, werden die allgemeinen Definitionen im folgenden Sinn gebraucht:
C₁-C₄-Alkyl bzw. C₁-C₆-Alkyl steht im allgemeinen für einen verzweigten oder unverzweigten Kohlenwasserstoffrest mit 1 bis 4 oder 6 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
   Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylproypyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2,-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl; Ethyl, Propyl, *iso*-Propyl, n-Butyl, 1-Methylpropyl, 2-Methylpropyl oder 1,1-Dimethylethyl.
   Aryl steht im allgemeinen für einen aromatischen Rest mit 6 Kohlenstoffatomen - auch in Zusammensetzungen, wobei der Aromat mit einer oder mehreren Niederalkylgruppe(n), Trifluormethylgruppe(n), Cyanogruppe(n), Alkoxygruppe(n), Nitrogruppe(n), Aminogruppe(n) und/oder einem oder mehreren Halogenatom(en) - untereinander gleich oder verschieden - substituiert sein kann; bevorzugter Arylrest ist ein gegebenenfalls substituierter Phenylrest, wobei als Substituenten Halogen - wie Fluor, Chlor oder Brom - sowie Hydroxyl bevorzugt sind.
   Alkoxy steht im allgemeinen für einen über ein Sauerstoffatom gebundenen geradkettigen oder verzweigten Kohlenwasserstoffrest mit 1 bis 6 Kohlenstoffatom(en). Bevorzugt ist ein Niederalkoxyrest mit 1 bis 3 Kohlenstoffatom(en). - Besonders bevorzugt ist die Methoxygruppe.

Die neuen Verbindungen können nach folgenden konventionellen Methoden hergestellt werden:
1. Umsetzung von Imidoestern der Formel II 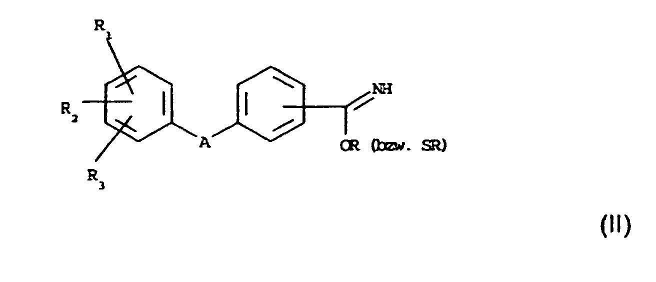 in der R₁ bis R₄, A und B die obige Bedeutung haben und R bevorzugt für einen C₁-C₆-Alkylrest oder für Benzyl steht (jedoch kann der Fachmann gewünschtenfalls auch Derivate anderer Alkohole einsetzen), und Ammoniak. Die Umsetzung erfolgt zweckmäßig in einem organischen Lösungsmittel bei Temperaturen zwischen etwa 0°C und der Siedetemperatur des Reaktionsgemischs, vorzugsweise zwischen Raumtemperatur und etwa 100°C bzw. der Siedetemperatur, soweit diese niedriger ist. Geeignete Lösungsmittel sind polare Lösungsmittel wie Methanol, Ethanol, Propanole.
   Bei hinreichend säurestabilen Ausgangsstoffen kann die Umsetzung statt über die Imidoester auch über die entsprechenden Säureimidchloride erfolgen.
2. Zur Herstellung von Verbindungen der Formel I, in denen A ein über O mit mindestens einem der Ringsysteme verknüpft ist:
   Umsetzung
      (a) eines Phenols der Formel III 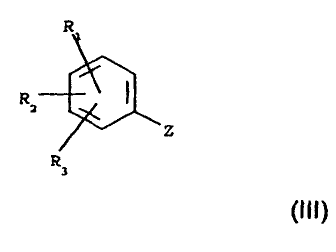 worin Z, OH darstellt und R₁, R₂ und R₃ die oben angegebene Bedeutung haben, mit einer Verbindung der allgemeinen Formel IV 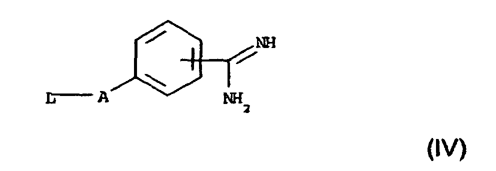 worin A die oben angegebene Bedeutung hat und L für eine nucleofuge Abgangsgruppe steht, bzw.
      (b) eines Phenols der Formel V 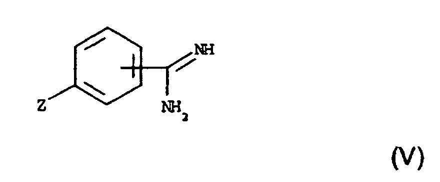 worin Z die oben angegebene Bedeutung hat, mit einer Verbindung der Formel VI: 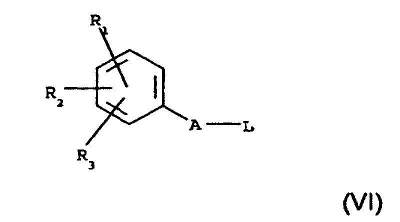 worin A, R₁, R₂, R₃ und L die oben angegebene Bedeutung haben.

      Die Umsetzung erfolgt in aprotischen Lösungsmitteln wie Dimethylsulfoxid, Dimethylformamid, Acetonitril oder Alkoholen wie Methanol, Ethanol oder Propanol unter Zusatz einer Base (Metallcarbonate, Metallhydroxide, Metallhydride) bei Temperaturen zwischen etwa 0 und 140°C bzw. der Siedetemperatur des Reaktionsgemischs.
      Die Phenole können auch in Form von Salzen, etwa der Alkalisalze, eingesetzt werden. Als nucleofuge Abgangsgruppe eignen sich z.B. Halogene, etwa Br oder Cl.
   3. Reduktion eines Amidoxims der Formel VII: 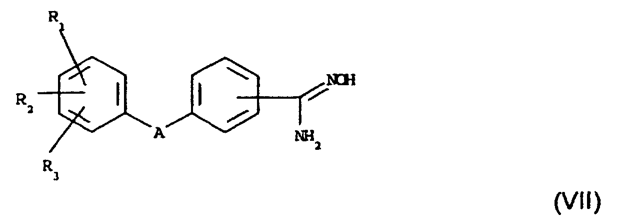
   worin A und R₁ bis R₃ die oben angegebene Bedeutung haben.

Für die Stufe der Reduktion des Amidoxims eignet sich die katalytische Hydrierung, insbesondere mit Raney-Nickel in einem niederen Alkohol, z. B. Methanol.

Zweckmäßig wird das Amidoxim der Formel unter Zugabe der berechneten Menge derjenigen Säure, deren Salz als Endprodukt gewünscht wird, in Methanol gelöst und bei Raumtemperatur unter leichtem Druck, z.B. bei 5 bar, bis zur beendeten Wasserstoffaufnahme hydriert.

Die Ausgangsstoffe können nach üblichen Methoden aus bekannten Verbindungen erhalten werden.

So können die Ausgangsstoffe für Verfahren 1 aus den entsprechenden Nitrilen durch Umsetzung mit HCl über die Stufe der Imidchloride bzw. direkt durch Umsetzung mit z.B. C₁-C₆-Alkoholen bzw. Benzylalkohol in Gegenwart einer Säure wie HCl erhalten werden. Auch die Umsetzung der Nitrile mit H₂S in Lösungsmitteln wie Pyridin oder Dimethylformamid in Gegenwart einer Base wie Triethylamin und anschließende Alkylierung bzw. Benzylierung führen zu Verbindungen der Formel II.

Ausgehend von Carbonsäureamiden, die im übrigen den Verbindungen der Formel II entsprechen, gelangt man auch durch Umsetzung mit einem Trialkytoxoniumsalz wie Triethyloxoniumtetrafluoroborat in einem Lösungsmittel wie Dichlormethan, Tetrahydrofuran oder Dioxan bei Temperaturen zwischen 0 und 50°C, vorzugweise bei Raumtemperatur zu Verbindungen der Formel II.
Für die Herstellung der Ausgangsstoffe der allgemeinen Formel VII können auch Umsetzungen entsprechender Amidoxime anstelle von Amidinen analog Verfahren 1 oder 2 dienen; durch analoge oder Umsetzung entsprechender Nitrile, aus denen abschließend durch Addition von Hydroxylamin die Ausgangsstoffe der allgemeinen Formel VII entstehen.

Wie gefunden wurde, zeichnen sich die Verbindungen der Formel I durch vielfältige Anwendungsmöglichkeiten auf therapeutischem Gebiet aus. Hervorzuheben sind solche Anwendungsmöglichkeiten, für welche die LTB₄-rezeptorantagonistischen Eigenschaften eine Rolle spielen. Hier sind insbesondere zu nennen: Arthiritis, Asthma, chronische obstruktive Lungenerkrankungen, etwa chronische Bronchitis, Psoriasis, Colitis ulcerosa, durch nichtsteroidale Antiphlogistika induzierte Gastro- oder Enteropathie, cystische Fibrose, Alzheimer-Krankheit, Schock, Reperfusionsschäden/Ischämien, Atherosklerose, Multiple Sklerose.

Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des LTB₄ oder eines anderen Moleküls (beispielsweise 12-HETE) mit dem LTB₄-Rezeptor einen Einfluß auf die Zell-Proliferation hat (etwa chronische myelozytische Leukämie).

Die neuen Verbindungen können auch in Kombination mit anderen Wirkstoffen angewendet werden, etwa solchen, die für dieselben Indikationen Verwendung finden, oder z.B. mit Antiallergika, Sekretolytika, β₂-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Zur pharmakologischen und biochemischen Untersuchung der Wirkungsverhältnisse eigenen sich Tests, wie sie beispielsweise in der WO 93/16036, S. 15 bis 17, - auf die hier inhaltlich Bezug genommen wird - dargestellt sind.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt.

Inhalationslösungen enthalten im allgemeinen zwischen etwa 0,5 und 5 % Wirkstoff. Die neuen Verbindungen können in üblichen Zubereitungen verabreicht werden, etwa als Tabletten, Dragees, Kapseln, Oblaten, Pulver, Granulate, Lösungen, Emulsionen, Sirupe, Inhalationsaerosole, Salben, Suppositorien.

Die nachstehenden Beispiele zeigen einige Möglichkeiten für die Formulierung der Darreichungsformen:

### Formulierungsbeispiele

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | . 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

### Synthesebeispiel

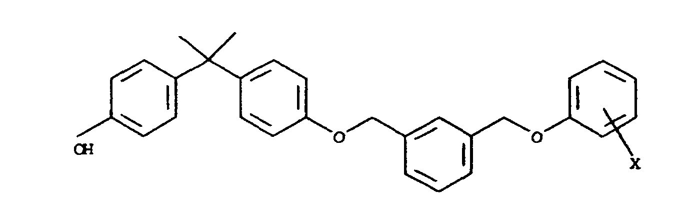

### Amidoxim: X = para-C(=NOH)NH₂

2,0 g des Nitrils der obigen Formel (X = *para*-CN) werden in 40 ml Ethanol vorgelegt, unter Rückfluß erhitzt und ein Gemsich aus 1 g Na₂CO₃ in 5 ml Wasser und 1,24 g Hydroxylamin x HCl zugetropft. Nach 5 h Erhitzen unter Rückfluß wird das Lösungsmittel abdestilliert, der Rückstand mit 50 ml Wasser verrührt, 3 x mit je 50 ml Essigester extrahiert und die vereinigten organischen Phasen getrocknet. Nach Filtriern wird im Vakuum eingeengt und der Rückstand mittels FlashChromatographie (Kieselgel 60, CH₂Cl₂/Methanol 9 : 1) gereinigt. Das Produkt wird in Ethanol gelöst, mit ethanolischer HCl angesäuert und mit Ether als Hydrochlorid ausgefällt. Das anfallende Öl wird mit Essigester kristallisiert. Ausbeute: 2,0 g weiße Kristalle.

### 4-[[3-[[4-[1-(4-Hydroxyphenyl)-1-methylethyl]phenoxy]methyl]phenyl]methoxy]-benzolcarboximidamid hydrochlorid (X = para-C(=NH)-NH₂)

2,0 g des Amidoxims der obigen Formel (X = *para*-C(=NOH)-NH₂) werden in 50 ml Methanol gelöst und mit 5 g methanolfeuchtem Raney-Nickel unter Zusatz von 1 ml 20 %iger Ammoniumchloridlösung 5 h bei Normaldruck und Raumtemperatur hydriert. Das Nickel wird abgesaugt und die Lösung über Kieselgur filtriert. Nach Einengen im Vakuum wird der Rückstand mit 50 ml Wasser verrührt. Die Kristalle werden abgesaugt und 2 x aus Ethanol/Ether umkristallisiert. Ausbeute: 1,0 g der Amidinverbindung (obige Formel, X = *para*-C(=NH)-NH₂) als Hydrochlorid, Fp. 234-236°C.

Gemäß dieser Vorschrift werden u. a. auch folgende Verbindungen erhalten:

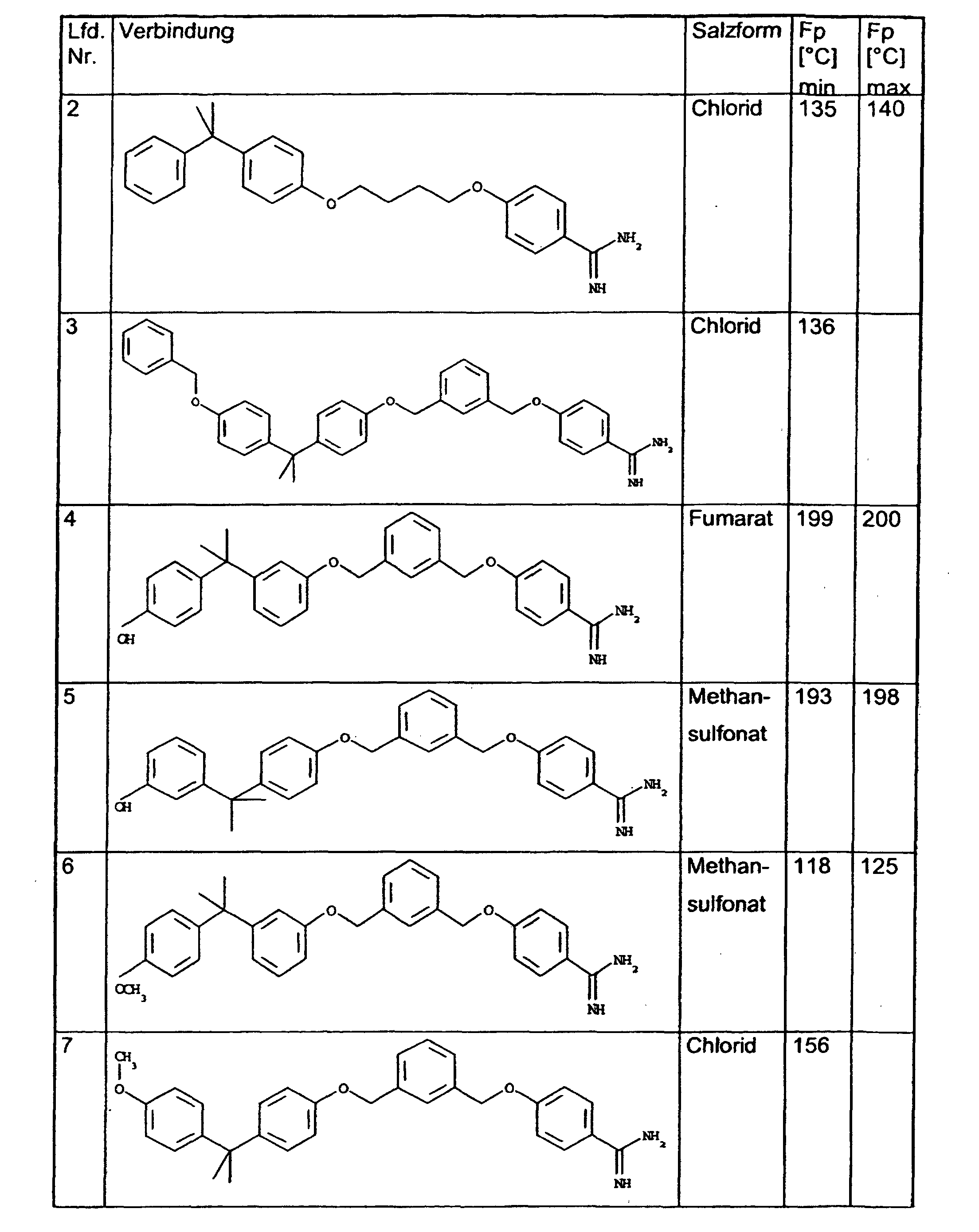

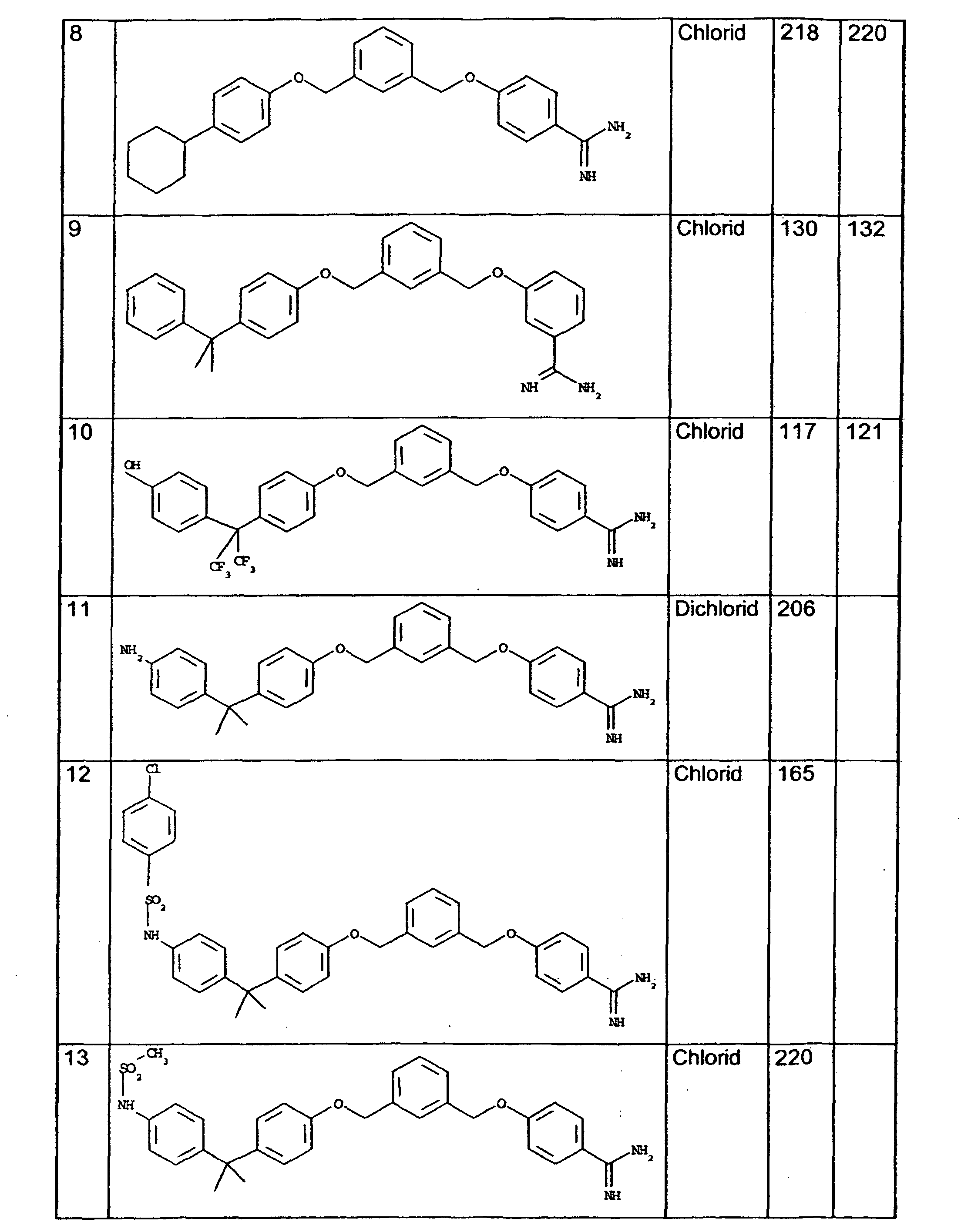

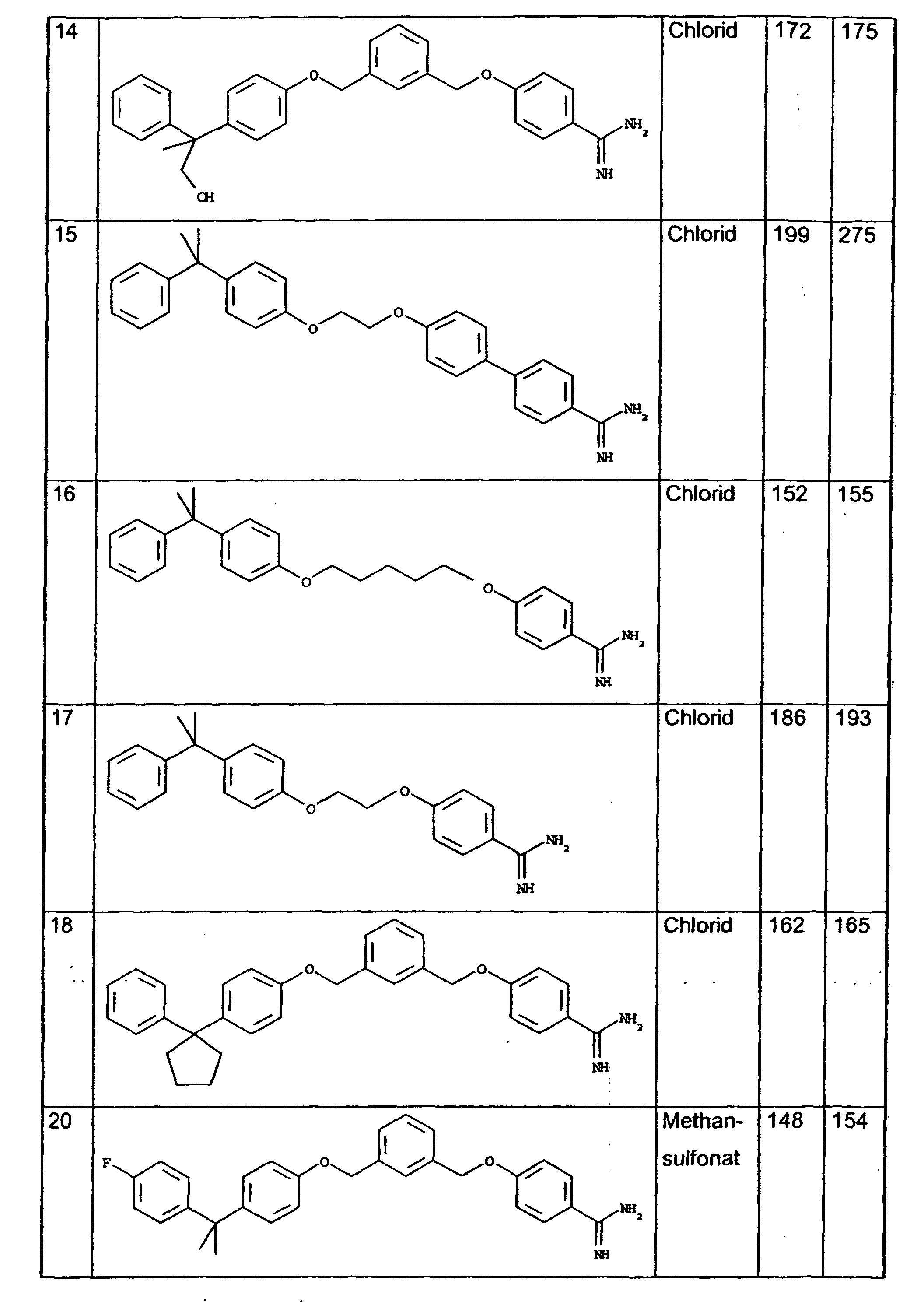

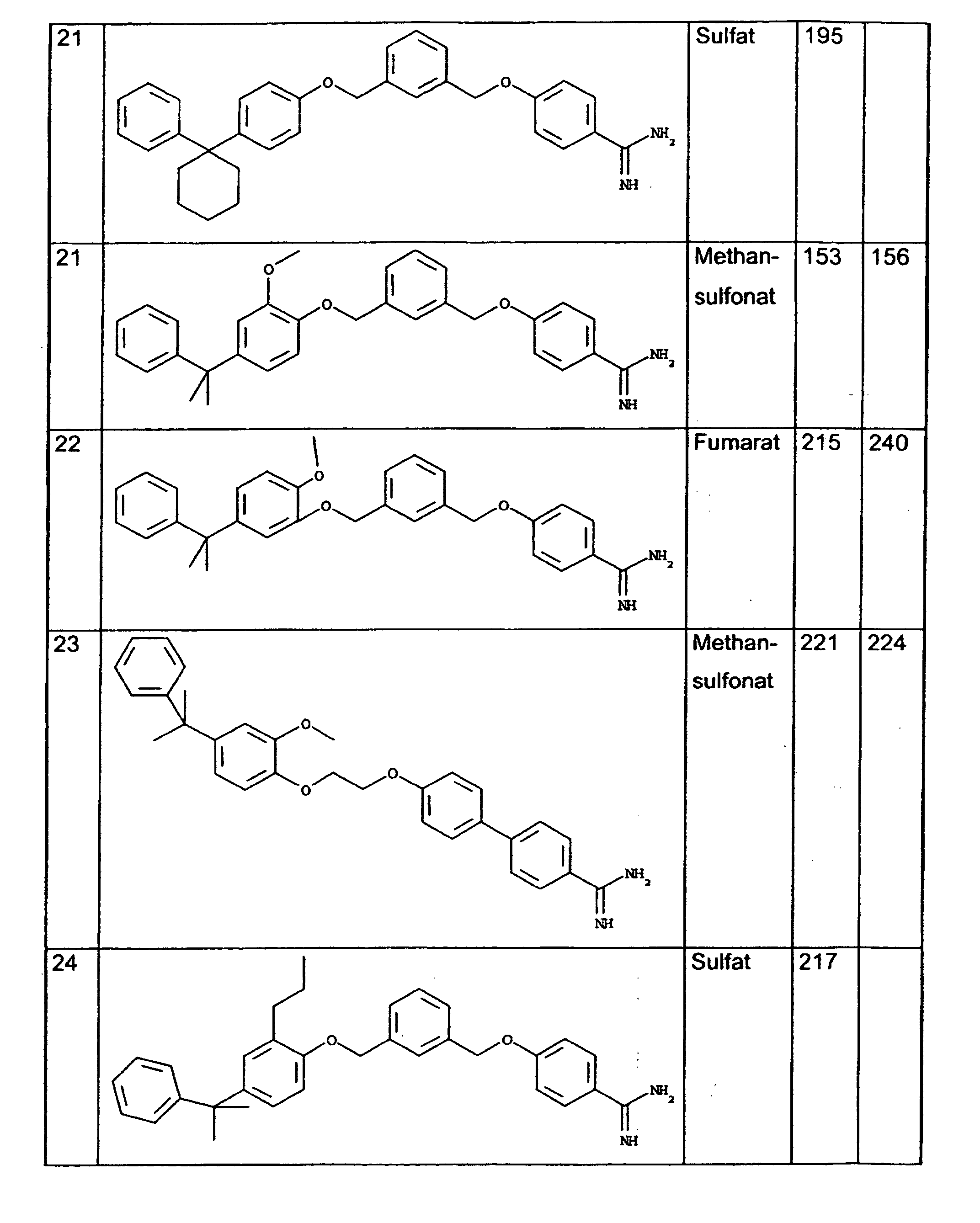

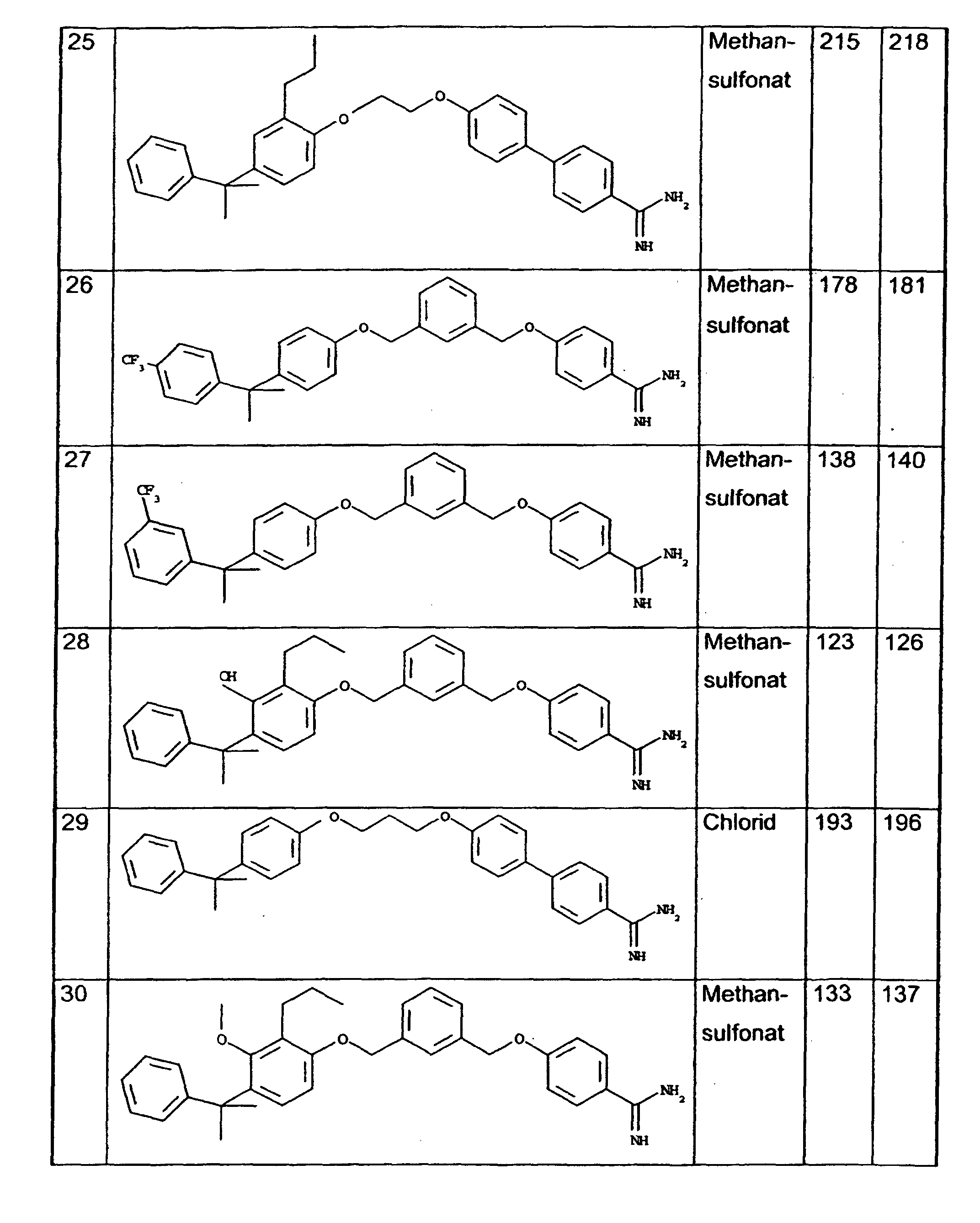

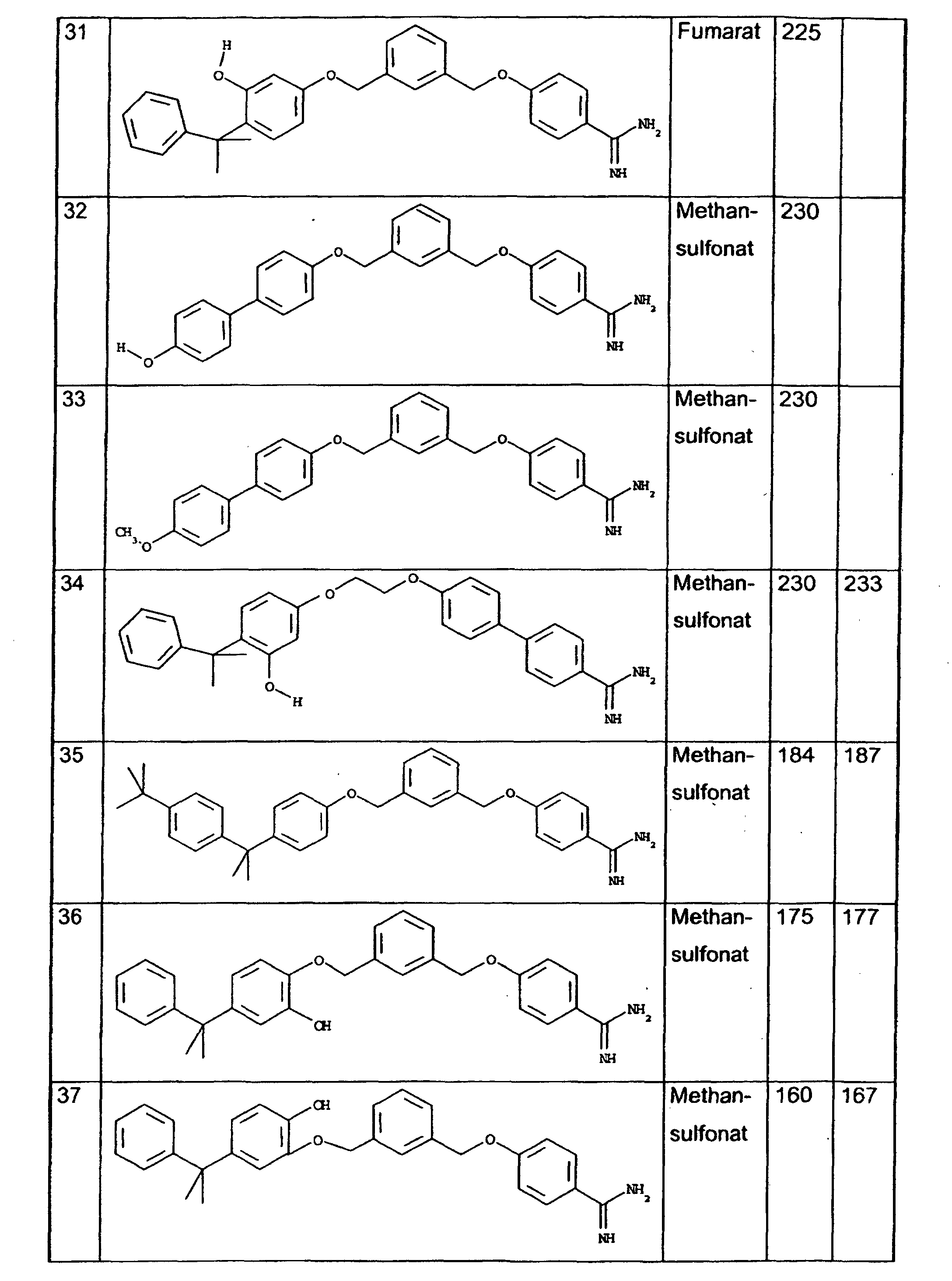

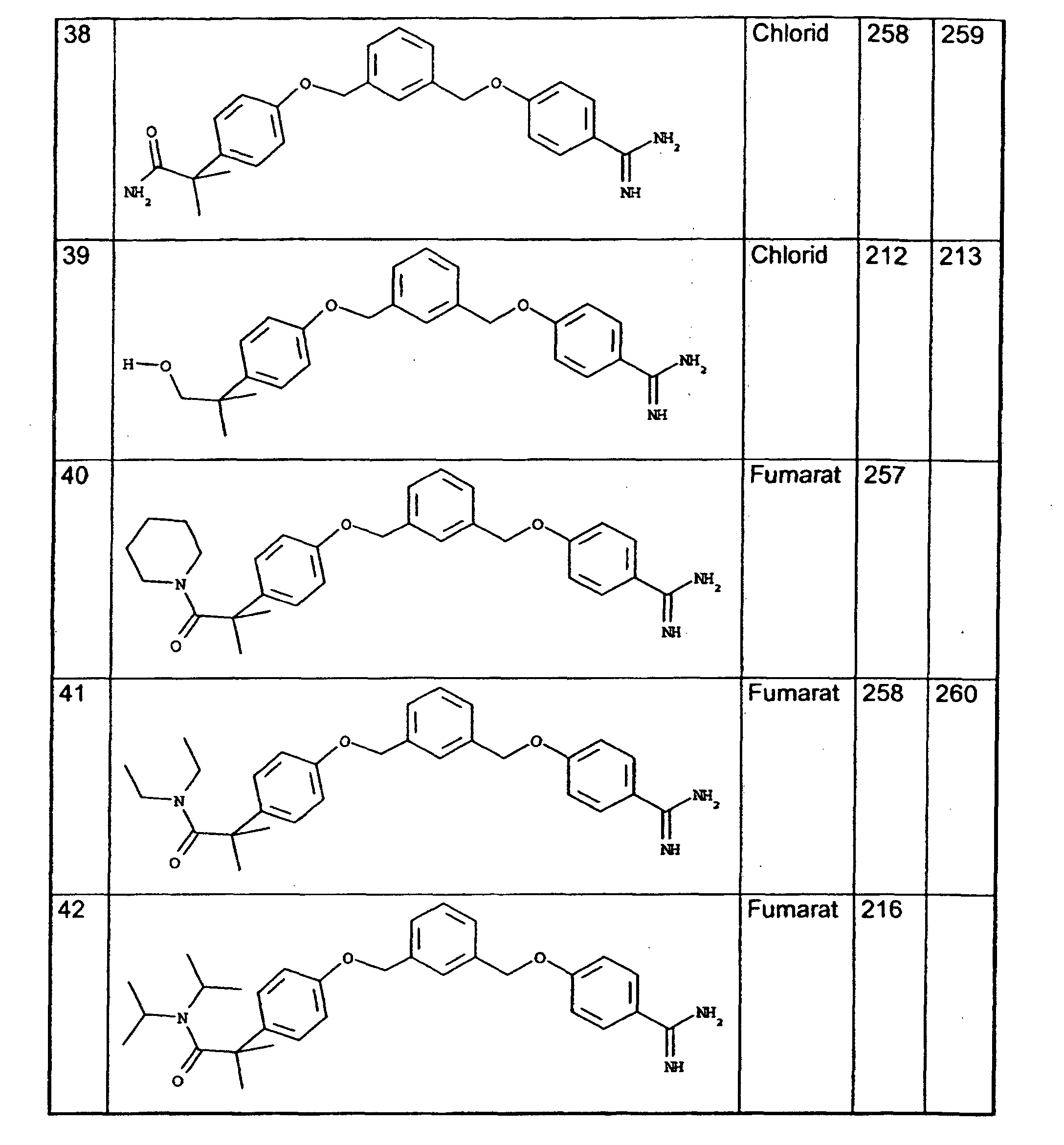

Überraschenderweise zeigen die in dem Beispiel und der Tabelle aufgeführten Verbindungen hervorragende Kᵢ-Werte, die zu einem großen Teil in einem Bereich von 0.2 bis 0.7 nmol/l liegen (RB.LTB4 / U937-Zellen).

## Patentansprüche

1. Verbindungen der allgemeinen Formel I 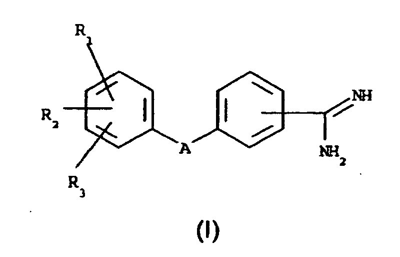 worin
A X₁-CₘH₂ₘ-X₂-, m eine ganze Zahl 2, oder 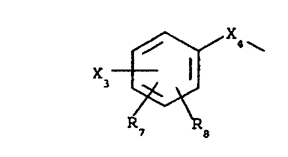
und
X₁ O;
X₂ 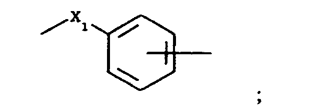
X₃ -X₁-CₙH₂ₙ-, worin n eine ganze Zahl 1 oder 2;
X₄ -CₙH₂ₙ-X₁-, worin n eine ganze Zahl 1 oder 2;
R₁ CR₄R₅Ar₃, C(CH₃)₂R₆;
R₂ H, C₁-C₆-Alkyl, OH, Cl, O-(C₁-C₆)-Alkyl;
R₃ H, C₁-C₆-Alkyl;
R₄ C₁-C₄-Alkyl, CF₃, CH₂OH;
R₅ H, C₁-C₄-Alkyl, CF₃, CH₂OH; oder
R₄ und R₅ auch gemeinsam eine C₄-C₆-Alkylengruppe bilden können;
R₆ CH₂OH, COOH, COO(C₁-C₄)-Alkyl, CONR₉R₁₀, CH₂NR₉R₁₀;
R₇ H, F, Cl, Br, OH, C₁-C₆-Alkyl oder C₁-C₆-Alkoxy;
R₈ H, F, Cl, Br, OH, C₁-C₆-Alkyl oder C₁-C₆-Alkoxy;
R₉ H, C₁-C₆-Alkyl;
R₁₀ H oder C₁-C₆-Alkyl; oder
R₉ und R₁₀ gemeinsam auch eine C₄-C₆-Alkylengruppe darstellen können;
Ar₃ einen gegebenenfalls durch ein oder mehrere Niederalkylgruppen mit 1 bis 4 Kohlenstoffatomen, Trifluormethylgruppen, Cyanogruppen, Alkoxygruppen mit 1 bis 8 Kohlenstoffatomen, Nitrogruppen, Hydroxygruppen, Aminogruppen und/oder einem oder mehreren Halogenatomen substituierten Arylrest mit 6 Kohlenstoffatomen
bedeuten können - mit der Maßgabe, daß
R₁ nicht die Bedeutung eines über eine C₁-C₄-Alkyleneinheit gebundenen unsubstituierten Phenylrestes haben darf; gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren.

2. Verbindungen gemäß der allgemeinen Formel I nach Anspruch 1, worin
A 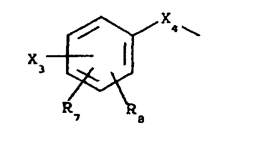
X₁ O;
und
X₃ -X₁-CH₂-;
X₄ -CH₂-X₁-;
R₁ CR₄R₅Ar₃, C(CH₃)₂R₆;
R₂ H, OH, O-(C₁-C₆)-Alkyl;
R₃ H;
R₄ CH₃, CH₂OH;
R₅ H, CH₃, CH₂OH; oder
R₄ und R₅ auch gemeinsam eine C₄-C₆-Alkylengruppe bilden können;
R₆ CH₂OH, COOH, COO(C₁-C₄)-Alkyl, CONR₉R₁₀, CH₂NR₉R₁₀;
R₇ H;
R₈ H;
R₉ H, C₁-C₆-Alkyl;
R₁₀ H oder C₁-C₆-Alkyl; oder
R₉ und R₁₀ gemeinsam auch eine C₄-C₆-Alkylengruppe darstellen können;
Ar₃ einen gegebenenfalls ein- oder mehrfach mit Hydroxy oder mit Hydroxy und C₁-C₆-Alkyl substituierten Arylrest;
bedeuten können -gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren.

3. 4-[[3-[[4-[1-(4-Hydroxyphenyl)-1-methylethyl]phenoxy]methyl]phenyl]methoxy]-benzolcarboximidamid hydrochlorid.

4. Verfahren zur Herstellung von Verbindungen gemäß der allgemeinen Formel I, **dadurch gekennzeichnet, daß** man einen Imidoester der allgemeinen Formel II 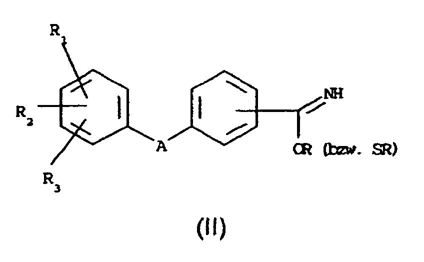 in der R₁ bis R₄ und A die in Anspruch 1 angegebene Bedeutung haben und R bevorzugt für einen C₁-C₆-Alkylrest oder für Benzyl steht mit Ammoniak, in einem organischen Lösungsmittel, vorzugsweise in einem polaren organischen Lösungsmittel, besonders bevorzugt in Methanol, Ethanol oder Propanolen bei Temperaturen zwischen etwa 0°C und der Siedetemperatur des Reaktionsgemischs, vorzugsweise zwischen Raumtemperatur und etwa 100°C bzw. der Siedetemperatur, soweit diese niedriger ist, umsetzt.

5. Verfahren nach Anspruch 4, **dadurch gekennzeichnet, daß** man als Ausgangsmaterial anstelle der Imidoester der allgemeinen Formel II die entsprechenden Säureimidchloride einsetzt.

6. Verfahren zur Herstellung von Verbindungen gemäß der allgemeinen Formel I, in denen A ein über O mit mindestens einem der Ringsysteme verknüpft ist, **dadurch gekennzeichnet, daß** man
ein Phenol der Formel III 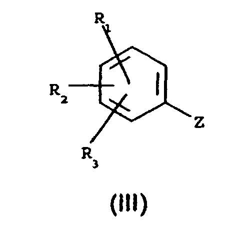 worin Z, OH darstellt und R₁, R₂ und R₃ die in Anspruch 1 angegebene Bedeutung haben, mit einer Verbindung der allgemeinen Formel IV 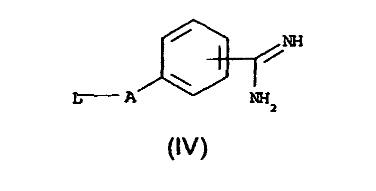 worin A die in Anspruch 1 angegebene Bedeutung hat und L für eine nucleofuge Abgangsgruppe steht, in aprotischen Lösungsmitteln wie Dimethylsulfoxid, Dimethylformamid, Acetonitril oder Alkoholen wie Methanol, Ethanol oder Propanol unter Zusatz einer Base, bevorzugt eines Metallcarbonats, Metallhydroxids oder Metallhydrids, bei Temperaturen zwischen 0 und 140°C bzw. der Siedetemperatur des Reaktionsgemischs, wobei die Phenole alternativ in Form ihrer Salze, bevorzugt der Alkalisalze, eingesetzt werden können und als nucleofuge Abgangsgruppe vorzugsweise ein Halogen und besonders bevorzugt Br oder Cl eingesetzt werden, umsetzt.

7. Verfahren zur Herstellung von Verbindungen der allgemeinen Formel I, in denen A ein über O mit mindestens einem der Ringsysteme verknüpft ist, **dadurch gekennzeichnet, daß** man
ein Phenol der allgemeinen Formel V 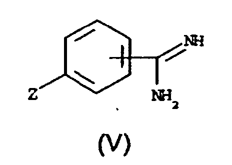 worin Z die in Anspruch 6 angegebene Bedeutung hat, mit einer Verbindung der Formel VI: 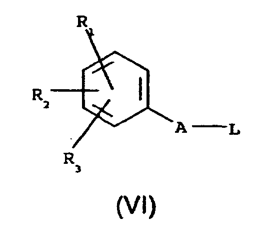 worin A, R₁, R₂, R₃ und L die in Anspruch 1 angegebene Bedeutung haben in aprotischen Lösungsmitteln wie Dimethylsulfoxid, Dimethylformamid, Acetonitril oder Alkoholen wie Methanol, Ethanol oder Propanol unter Zusatz einer Base, bevorzugt eines Metallcarbonats, Metallhydroxids oder Metallhydrids, bei Temperaturen zwischen 0 und 140°C bzw. der Siedetemperatur des Reaktionsgemischs, wobei die Phenole alternativ in Form ihrer Salze, bevorzugt der Alkalisalze, eingesetzt werden können und als nucleofuge Abgangsgruppe vorzugsweise ein Halogen und besonders bevorzugt Br oder Cl eingesetzt werden, umsetzt.

8. Verfahren zur Herstellung von Verbindungen gemäß der allgmeinen Formel I, **dadurch gekennzeichnet, daß** ein Amidoxim der allgemeinen Formel VII 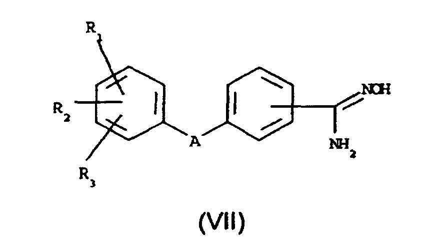 worin A und R₁ bis R₃ die in Anspruch 1 angegebene Bedeutung haben, bevorzugt auf katalytischem Wege, besonders bevorzugt in Gegenwart von Raney-Nickel, in einem inerten polaren Lösungsmittel, vorzugsweise in einem niederen Alkohol, besonders bevorzugt in Methanol, unter einem erhöhten Druck besonders bevorzugt bei einem Druck von 5 bar, reduziert

9. Pharmazeutische Zubereitung, **gekennzeichnet durch** einen Gehalt an einer Verbindung nach einem der Ansprüche 1 bis 3 und deren Säureadditionssalze neben üblichen Hilfs- und Trägersstoffen.

10. Verbindung nach einem der Ansprüche 1 bis 3 zur Verwendung als Arzneimittel.

11. Verwendung von Verbindungen nach einem der Ansprüche 1 bis 3 zur Herstellung eines Arzneimittel mit LTB₄-antagonistischer Wirkung.

12. Verwendung von Verbindungen nach einem der Ansprüche 1 bis 3 zur Herstellung eines Medikaments zur therapeutischen Behandlung von Arthiritis, Asthma, chronischer obstruktiver Lungenerkrankung wie chronischer Bronchitis, Psoriasis, Colitis ulcerosa, durch nichtsteroidale Antiphlogistika induzierter Gastro- oder Enteropathie, cystischer Fibrose, Alzheimer-Krankheit, Schock, Reperfusionsschäden/Ischämien, Atherosklerose, multipler Sklerose.

## Claims

1. Compounds of general formula I 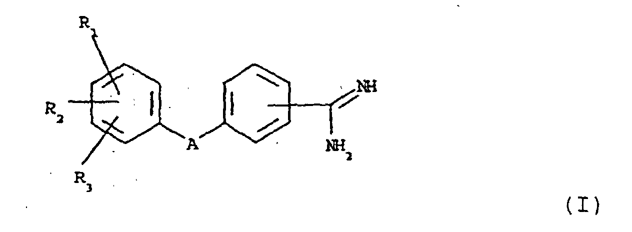 wherein
A denotes X₁-CₘH₂ₘ-X₂, m is an integer 2, or 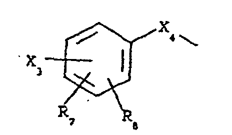
and
X₁ denotes O;
X₂ is 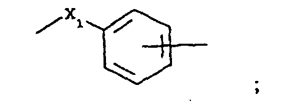
X₃ denotes X₁-CₙH₂ₙ wherein n is the integer 1 or 2;
X₄ denotes CₙH₂ₙ-X₁ wherein n is the integer 1 or 2;
R₁ denotes CR₄R₅Ar₃, C(CH₃)₂R₆;
R₂ denotes H, C₁₋₆-alkyl, OH, Cl, O-(C₁₋₆)-alkyl;
R₃ denotes H, C₁₋₆-alkyl;
R₄ denotes C₁₋₄-alkyl, CF₃, CH₂OH;
R₅ denotes H, C₁₋₄-alkyl, CF₃, CH₂OH; and
R₄ and R₅ together may also form a C₄₋₆-alkylene group;
R₆ denotes CH₂OH, COOH, COO(C₁₋₄)alkyl, CONR₉R₁₀, CH₂NR₉R₁₀;
R₇ denotes H, F, Cl, Br, OH, C₁₋₆-alkyl or C₁₋₆-alkoxy;
R₈ denotes H, F, Cl, Br, OH, C₁₋₆-alkyl or C₁₋₆-alkoxy;
R₉ denotes H, C₁₋₆-alkyl;
R₁₀ denotes H or C₁₋₆-alkyl and
R₉ and R₁₀ together may also represent a C₄₋₆-alkylene group;
Ar₃ denotes an aryl group with 6 carbon atoms optionally substituted with one or more lower alkyl groups having 1 to 4 carbon atoms, trifluoromethyl groups, cyano groups, alkoxy groups having 1 to 8 carbon atoms, nitro groups, amino groups and/or one or more halogen atoms,
with the proviso that
R₁ cannot represent an unsubstituted phenyl group bound via a C₁₋₄-alkylene unit;
optionally in the form of the individual optical isomers, mixtures of the individual enantiomers or racemates and in the form of the free bases or the corresponding acid addition salts with pharmacologically acceptable acids.

2. Compounds according to general formula I according to claim 1, wherein
A denotes 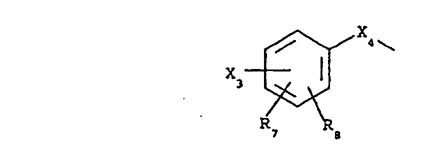
and
X₁ is O;
X₃ denotes X₁-CH₂;
X₄ denotes CH₂-X₁;
R₁ denotes CR₄R₅Ar₃, C(CH₃)₂R₆;
R₂ denotes H, OH, O-(C₁₋₆)-alkyl;
R₃ denotes H;
R₄ denotes CH₃, CH₂OH;
R₅ denotes H, CH₃, CH₂OH; and
R₄ and R₅ together may also denote a C₄₋₆-alkylene group;
R₆ denotes CH₂OH, COOH, COO(C₁₋₄) -alkyl, CONR₉R₁₀, CH₂NR₉R₁₀;
R₇ denotes H;
R₈ denotes H;
R₉ denotes H, C₁₋₆-alkyl ;
R₁₀ denotes H or C₁₋₆-alkyl; and
R₉ and R₁₀ together may also denote a C₄₋₆-alkylene group;
Ar₃ denotes an aryl group optionally mono- or polysubstituted by hydroxy or by hydroxy and C₁₋₆-alkyl ;
optionally in the form of the individual optical isomers, mixtures of the individual enantiomers or racemates and in the form of the free bases or the corresponding acid addition salts with pharmacologically acceptable acids.

3. 4-[[3-[[4-[1-(4-Hydroxyphenyl)-1-methylethyl]-phenoxy]methyl]phenyl]methoxy]benzenecarboximidamide hydrochloride.

4. Process for preparing compounds according to general formula I, **characterised in that** an imido ester of general formula II 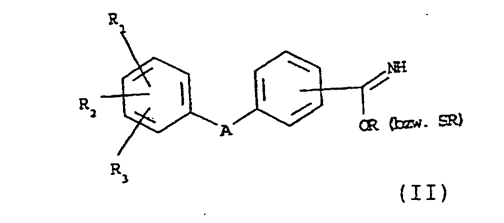 wherein R₁ to R₄ and A are defined as in claim 1 and R preferably represents a C₁₋₆-alkyl group or benzyl, is reacted with ammonia in an organic solvent, preferably in a polar organic solvent, most preferably in methanol, ethanol or a propanol at temperatures between about 0°C and the boiling temperature of the reaction mixture, preferably between ambient temperature and about 100°C, or the boiling temperature, if this is lower.

5. Process according to claim 4, **characterised in that**, instead of the imido esters of general formula II, the corresponding acid imide chlorides are used as starting material.

6. Process for preparing compounds according to general formula I, wherein A is linked to at least one of the ring systems via O, **characterised in that**
a phenol of formula III 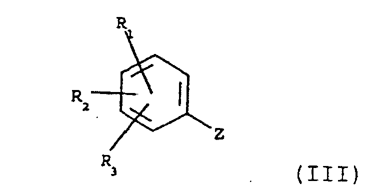 wherein Z denotes OH and R₁, R₂ and R₃ are as defined in claim 1, is reacted with a compound of general formula IV 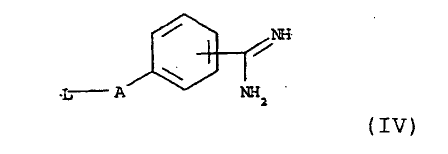 wherein A is defined as in claim 1 and L denotes a nucleofugic leaving group, in aprotic solvents such as dimethylsulphoxide, dimethylformamide, acetonitrile or alcohols such as methanol, ethanol or propanol with the addition of a base, preferably a metal carbonate, metal hydroxide or metal hydride, at temperatures between 0 and 140°C or the boiling temperature of the reaction mixture, whilst the phenols may alternatively be used in the form of their salts, preferably the alkali metal salts, and the nucleofugic leaving group used is preferably a halogen and most preferably Br or Cl.

7. Process for preparing compounds of general formula I wherein A is linked to at least one of the ring systems via O, **characterised in that**
a phenol of general formula V 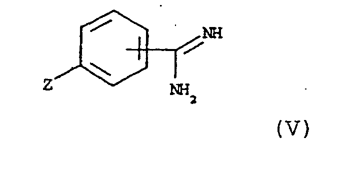 wherein Z is defined as in claim 6, is reacted with a compound of formula VI 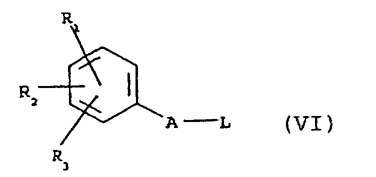 wherein A, R₁, R₂, R₃ and L are defined as in claim 1, in aprotic solvents such as dimethylsulphoxide, dimethylformamide, acetonitrile or alcohols such as methanol, ethanol or propanol with the addition of a base, preferably a metal carbonate, metal hydroxide or metal hydride, at temperatures between 0 and 140°C or the boiling temperature of the reaction mixture, whilst the phenols may alternatively be used in the form of their salts, preferably the alkali metal salts, and the nucleofugic leaving group used is preferably a halogen and most preferably Br or Cl.

8. Process for preparing compounds according to general formula I, **characterised in that** an amidoxime of general formula VII 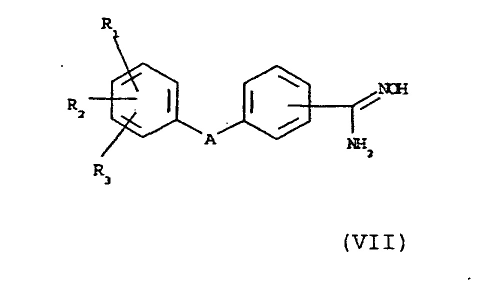 wherein A and R₁ to R₃ are defined as in claim 1, is reduced, preferably by a catalytic method, most preferably in the presence of Raney nickel, in an inert polar solvent, preferably in a lower alcohol, most preferably in methanol, under elevated pressure, most preferably at a pressure of 5 bar.

9. Pharmaceutical preparation, **characterised in that** it contains a compound according to one of claims 1 to 3 and the acid addition salts thereof together with conventional excipients and carriers.

10. Compound according to one of claims 1 to 3, for use as a medicament.

11. Use of compounds according to one of claims 1 to 3, for preparing a medicament with an LTB₄-antagonistic activity.

12. Use of compounds according to one of claims 1 to 3, for preparing a medicament for the therapeutic treatment of arthritis, asthma, chronic obstructive lung disease such as chronic bronchitis, psoriasis, ulcerative colitis, gastropathy or enteropathy induced by non-steroidal anti-inflammatories, cystic fibrosis, Alzheimer's disease, shock, reperfusion damage/ischaemia, atherosclerosis and multiple sclerosis.

## Revendications

1. Composés de formule générale I 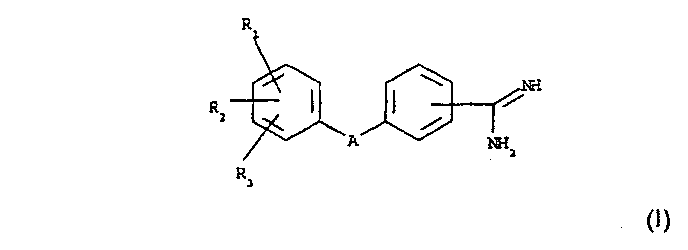 où
A peut représenter X₁-CₘH₂ₘ-X₂-, m peut représenter un nombre entier 2, ou 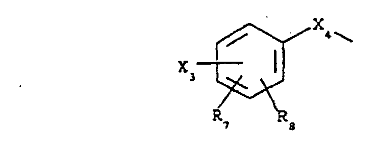 et
X₁ peut représenter O ;
X₂ peut représenter 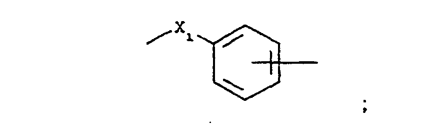 X₃ peut représenter -X₁-CₙH₂ₙ-, où n peut représenter un nombre entier 1 ou 2 ;
X₄ peut représenter -CₙH₂ₙ-X₁-, où n peut représenter un nombre entier 1 ou 2 ;
R₁ peut représenter CR₄R₅Ar₃, C(CH₃)₂R₆ ;
R₂ peut représenter H, alkyle en C₁-C₆, OH, Cl, O-alkyle en C₁-C₆ ;
R₃ peut représenter H, alkyle en C₁-C₆ ;
R₄ peut représenter alkyle en C₁-C₄, CF₃, CH₂OH ;
R₅ peut représenter H, alkyle en C₁-C₄, CF₃, CH₂OH ; ou
R₄ et R₅ peuvent aussi former en commun un groupe alkylène en C₄-C₆ ;
R₆ peut représenter CH₂OH, COOH, COO-alkyle en C₁-C₄, CONR₉R₁₀, CH₂NR₉R₁₀ ;
R₇ peut représenter H, F, Cl, Br, OH, alkyle en C₁-C₆ ou alcoxy en C₁-C₆ ;
R₈ peut représenter H, F, Cl, Br, OH, alkyle en C₁-C₆ ou alcoxy en C₁-C₆ ;
R₉ peut représenter H, alkyle en C₁-C₆ ;
R₁₀ peut représenter H ou alkyle en C₁-C₆ ; ou
R₉ et R₁₀ peuvent aussi représenter en commun un groupe alkylène en C₄-C₆ ;
Ar₃ peut représenter un reste aryle ayant 6 atomes de carbone éventuellement substitué par un ou plusieurs groupes alkyle inférieurs ayant 1 à 4 atomes de carbone, groupes trifluorométhyle, groupes cyano, groupes alcoxy ayant 1 à 8 atomes de carbone, groupes nitro, groupes hydroxyle, groupes amino et/ou un ou plusieurs atomes d'halogène
avec la condition que
R₁ ne peut pas avoir la signification d'un reste phényle non substitué lié par le biais d'une unité alkylène en C₁-C₄ ;
éventuellement sous forme des différents isomères optiques, de mélanges des différents énantiomères ou racémates et sous forme des bases libres ou des sels d'addition d'acide correspondants avec des acides pharmacologiquement irréprochables.

2. Composés de formule générale I selon la revendication 1 où
A peut représenter 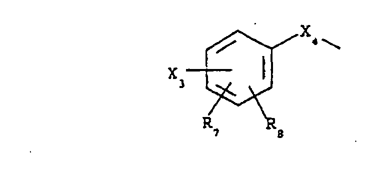 et
X₁ peut représenter O ;
X₃ peut représenter -X₁-CH₂- ;
X₄ peut représenter -CH₂-X₁- ;
R₁ peut représenter CR₄R₅Ar₃, C(CH₃)₂R₆ ;
R₂ peut représenter H, OH, O-alkyle en C₁-C₆ ;
R₃ peut représenter H ;
R₄ peut représenter CH₃, CH₂OH ;
R₅ peut représenter H, CH₃, CH₂OH ; ou
R₄ et R₅ peuvent aussi former en commun un groupe alkylène en C₄-C₆ ;
R₆ peut représenter CH₂OH, COOH, COO-alkyle en C₁-C₄, CONR₉R₁₀, CH₂NR₉R₁₀ ;
R₇ peut représenter H ;
R₈ peut représenter H ;
R₉ peut représenter H, alkyle en C₁-C₆ ;
R₁₀ peut représenter H ou alkyle en C₁-C₆ ; ou
R₉ et R₁₀ peuvent aussi représenter en commun un groupe alkylène en C₄-C₆ ;
Ar₃ peut représenter un reste aryle éventuellement substitué une ou plusieurs fois par hydroxyle ou par hydroxyle et alkyle en C₁-C₆ ;
éventuellement sous forme des différents isomères optiques, de mélanges des. différents énantiomères ou racémates et sous forme des bases libres ou des sels d'addition d'acide correspondants avec des acides pharmacologiquement irréprochables.

3. Chlorhydrate de 4-[[3-[[4-[1-(4-hydroxyphényl)-1-méthyléthyl]phénoxy]méthyl]phényl]méthoxy]-benzènecarboximidamide.

4. Procédé de préparation de composés selon la formule générale I **caractérisé en ce que** l'on fait réagir un imidoester de formule générale II 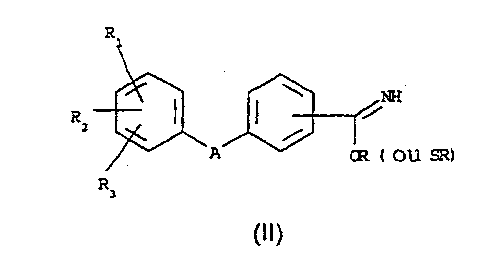 où R₁ à R₄ et A ont la signification indiquée dans la revendication 1 et R représente de préférence un reste alkyle en C₁-C₆ ou benzyle avec l'ammoniac, dans un solvant organique, de préférence dans un solvant organique polaire, de manière particulièrement préférée dans le méthanol, l'éthanol ou les propanols à des températures entre environ 0°C et la température d'ébullition du mélange réactionnel, de préférence entre la température ambiante et environ 100°C ou la température d'ébullition, dans la mesure où celle-ci est plus basse.

5. Procédé selon la revendication 4 **caractérisé en ce que** l'on utilise comme produit de départ à la place de l'imidoester de formule générale II les chlorures d'imide d'acide correspondants.

6. Procédé de préparation de composés selon la formule générale I où A est lié par le biais de O à au moins l'un des systèmes cycliques, **caractérisé en ce que** l'on fait réagir
un phénol de formule III 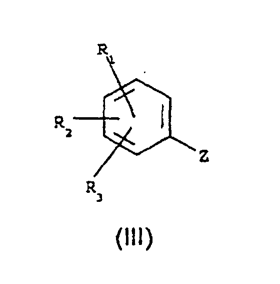 où Z représente OH et R₁, R₂ et R₃ ont la signification indiquée dans la revendication 1, avec un composé de formule générale IV 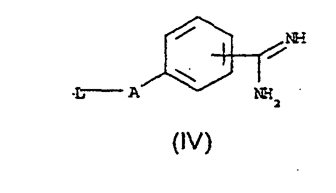 où A a la signification indiquée dans la revendication 1 et L représente un groupe partant nucléofuge, dans des solvants aprotiques comme le diméthylsulfoxyde, le diméthylformamide, l'acétonitrile ou des alcools comme le méthanol, l'éthanol ou le propanol avec addition d'une base, de préférence un carbonate métallique, un hydroxyde métallique ou un hydrure métallique, à des températures entre 0 et 140°C ou la température d'ébullition du mélange réactionnel, où les phénols peuvent être utilisés à titre d'alternative sous forme de leurs sels, de préférence les sels alcalins, et de préférence un halogène et de manière particulièrement préférée Br ou Cl est utilisé comme groupe partant nucléofuge.

7. Procédé de préparation de composés de formule générale I où A est lié par le biais de O à au moins l'un des systèmes cycliques, **caractérisé en ce que** l'on fait réagir un phénol de formule générale V 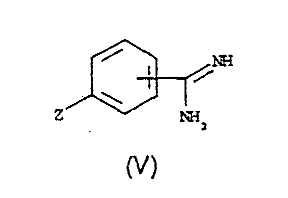 où Z a la signification indiquée dans la revendication 6 avec un composé de formule VI : 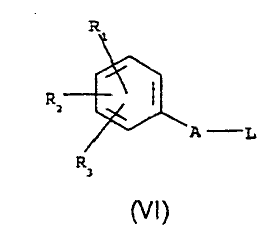 où A, R₁, R₂, R₃ et L ont la signification indiquée dans la revendication 1, dans des solvants aprotiques comme le diméthylsulfoxyde, le diméthylformamide, l'acétonitrile ou des alcools comme le méthanol, l'éthanol ou le propanol avec addition d'une base, de préférence un carbonate métallique, un hydroxyde métallique ou un hydrure métallique, à des températures entre 0 et 140°C ou la température d'ébullition du mélange réactionnel, où les phénols peuvent être utilisés à titre d'alternative sous forme de leurs sels, de préférence les sels alcalins, et de préférence un halogène et de manière particulièrement préférée Br ou Cl est utilisé comme groupe partant nucléofuge.

8. Procédé de préparation de composés selon la formule générale I **caractérisé en ce qu'**une amidoxime de formule générale VII 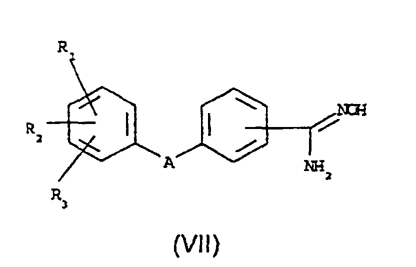 où A et R₁ à R₃ ont la signification indiquée dans la revendication 1, est réduite de préférence par voie catalytique, de manière particulièrement préférée en présence de nickel de Raney, dans un solvant polaire inerte, de préférence dans un alcool inférieur, de manière particulièrement préférée dans le méthanol, sous pression, de manière particulièrement préférée à une pression de 5 bar.

9. Préparation pharmaceutique **caractérisée par** une teneur en un composé selon l'une des revendications 1 à 3 et ses sels d'addition d'acide, outre des adjuvants et supports courants.

10. Composé selon l'une des revendications 1 à 3 destiné à être utilisé comme médicament.

11. Utilisation de composés selon l'une des revendications 1 à 3 pour la production d'un médicament à effet antagoniste de LTB₄.

12. Utilisation de composés selon l'une des revendications 1 à 3 pour la production d'un médicament pour le traitement thérapeutique de l'arthrite, de l'asthme, des maladies pulmonaires obstructives chroniques comme la bronchite chronique, du psoriasis, de la colite ulcéreuse, de la gastro- ou entéropathie induite par des antiphlogistiques non stéroïdiens, de la fibrose kystique, de la maladie d'Alzheimer, du choc, des lésions de reperfusion/ischémies, de l'athérosclérose, de la sclérose en plaques.
